(19) Europäisches Patentamt
European Patent Office
Office européen des brevets

(11) EP 4 005 597 A1

(12) EUROPEAN PATENT APPLICATION
published in accordance with Art. 153(4) EPC

(43) Date of publication:
01.06.2022 Bulletin 2022/22

(21) Application number: 20847509.5

(22) Date of filing: 22.07.2020

(51) International Patent Classification (IPC):
A61K 45/00 (2006.01)     A61P 19/00 (2006.01)
C12N 5/0735 (2010.01)    C12N 5/0775 (2010.01)
C12N 5/10 (2006.01)      C12N 11/04 (2006.01)
A61K 35/28 (2015.01)     A61K 35/545 (2015.01)

(52) Cooperative Patent Classification (CPC):
A61K 35/28; A61K 35/545; A61K 45/00;
A61P 19/00; C12N 5/10; C12N 11/04

(86) International application number:
PCT/JP2020/028406

(87) International publication number:
WO 2021/020268 (04.02.2021 Gazette 2021/05)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 26.07.2019 JP 2019137482

(71) Applicant: FUJIFILM Corporation
Tokyo 106-8620 (JP)

(72) Inventors:
• HIRATSUKA Takahiro
  Ashigarakami-gun, Kanagawa 258-8577 (JP)
• HONDA Masaki
  Nagoya-shi, Aichi 464-8650 (JP)
• ITO Masaaki
  Nagoya-shi, Aichi 464-8650 (JP)
• AKIYAMA Yasunori
  Nagoya-shi, Aichi 464-8650 (JP)

(74) Representative: Hoffmann Eitle
Patent- und Rechtsanwälte PartmbB
Arabellastraße 30
81925 München (DE)

(54) BIOGRAFT MATERIAL

(57) An object of the present invention is to provide a bone regenerative agent showing a high bone regeneration effect. According to the present invention, a bone regenerative agent containing gelatin-containing granules and stem cells is provided. Gelatin preferably has repetitions of a sequence represented by Gly-X-Y, which is characteristic of collagen. Here, a plurality of Gly-X-Y's may be the same as or different from each other, and in the formula, X and Y each independently represent any of amino acid. A molecular weight of the gelatin is preferably 2 KDa or more and 100 KDa or less.

FIG. 1

**Description**

Field of the Invention

The present invention relates to a bone regenerative agent containing gelatin-containing granules and a stem cell.

Background Art

As a substrate for regenerative medicine in the fields of dentistry, oral surgery, orthopedics, and the like, a substrate using gelatin having high bioaffinity as a raw material is known.

Patent Document 1 discloses a bone regenerative agent and a bone supplement preparation containing recombinant gelatin. In the bone regenerative agent and the bone supplement preparation described in Patent Document 1, the supplement material carrier itself can promote bone regeneration. A shape of gelatin disclosed in Examples of Patent Document 1 is a gel, and a site where bone regeneration can be confirmed is a bone defect part formed in the parietal bone of a rat (Example 4) and a tooth extraction site formed in the oral cavity of a beagle dog (Example 7).

Patent Document 2 discloses a cartilage regeneration material containing a mosaic cell mass using a petal-shaped block of recombinant gelatin and mesenchymal stem cells. A site where cartilage regeneration can be confirmed in Examples of Patent Document 2 is a rabbit osteochondral defect site (Example 1).

Prior Art Documents

Patent Documents

Patent Document 1: WO2011/027850A
Patent Document 2: WO2016/148245A

**SUMMARY OF THE INVENTION**

It has been confirmed that a regenerative medicine substrate using a recombinant gelatin as a raw material has a certain bone regeneration effect and cartilage regeneration effect. However, a place where a tissue regeneration can be confirmed in practice is limited to a place where a bone tissue or a cartilage tissue originally exists, such as a bone defect part formed in the parietal bone. It is presumed that such a place is relatively easy to regenerate a bone tissue and a cartilage tissue.

On the other hand, cleft jaw (gnathoschisis) is a condition in which gums have a fissure and is one of congenital anomalies. In order to fill the cleft jaw, it is necessary to supplement a bone tissue, and an autotransplantation method of a hipbone is known. However, since autologous bone transplanting from the hipbone or the like imposes a heavy burden on a patient, an alternative treatment method is required. It has not been sufficiently studied whether a regenerative medicine substrate using recombinant gelatin as a raw material exhibits a sufficient bone regeneration effect even in a place where there is originally no bone, such as reconstruction of a cleft jaw part in the treatment of cleft lip and palate.

As described above, from the viewpoint of reducing the burden on patients, there has been a demand for development of a regenerative medicine substrate that exhibits a tissue regeneration effect more quickly. An object of the present invention is to provide a bone regenerative agent showing a high bone regeneration effect.

As a result of intensive studies to achieve the above object, the present inventors have found that a high bone regeneration rate can be achieved by using gelatin-containing granules and a stem cell in combination. The present invention has been completed based on the finding.

That is, according to the present invention, the following aspects of the invention are provided.

<1> A bone regenerative agent comprising:

gelatin-containing granules; and
stem cells.

<2> The bone regenerative agent according to <1>,

in which gelatin has repetitions of a sequence represented by Gly-X-Y, which is characteristic of collagen,

where a plurality of Gly-X-Y's may be the same as or different from each other, and in the formula, X and Y each independently represent any of amino acid, and

a molecular weight of the gelatin is 2 KDa or more and 100 KDa or less.

<3> The bone regenerative agent according to <1> or <2>,
in which a size of one gelatin-containing granule is 100 μm or more and 2000 μm or less.
<4> The bone regenerative agent according to any one of <1> to <3>,
in which the stem cells are mesenchymal stem cells, iPS cells, or ES cells.
<5> The bone regenerative agent according to any one of <1> to <4>,
in which the stem cells are adipose-derived stem cells, bone marrow-derived stem cells, or dental pulp-derived stem cells.
<6> The bone regenerative agent according to any one of <1> to <5>,
in which the number of stem cells per 1 mg of the gelatin-containing granules is $1.0 \times 10^3$ or more.
<7> The bone regenerative agent according to any one of <1> to <6>,
in which 70% or more of the stem cells are attached to a surface or a pore of the surface of the gelatin-containing granules.
<8> The bone regenerative agent according to any one of <1> to <7>, which is used for a cleft jaw part.

<A> A bone regeneration method comprising:
administering a bone regenerative agent containing gelatin-containing granules and stem cells to a patient.
<B> A therapeutic agent, which is used for a bone regeneration treatment, comprising:

gelatin-containing granules; and
stem cells.

<C> Use of a combination of gelatin-containing granules and stem cells, for producing a bone regenerative agent.

According to the bone regenerative agent according to an embodiment of the present invention, a high bone regeneration effect can be exhibited.

**BRIEF DESCRIPTION OF THE DRAWINGS**

Fig. 1 is an image of granules + stem cells (left) and petal-shaped blocks + stem cells (right).
Fig. 2 shows measurement results of changes in bone volume over time in each transplantation group.
Fig. 3 is an image of a transplantation part.
Fig. 4 shows measurement results of changes in bone volume over time in each transplantation group.
Fig. 5 shows CT images (typical examples) of an affected area in each transplantation group.

**DESCRIPTION OF THE PREFERRED EMBODIMENTS**

Hereinafter, embodiments of the present invention will be described in detail.
A bone regenerative agent according to the embodiment of the present invention comprises gelatin-containing granules and stem cells.
The bone regenerative agent according to the embodiment of the present invention can improve a bone regeneration rate as demonstrated in a rat parietal bone defect model in examples described later. In addition, according to the bone regenerative agent according to the embodiment of the present invention, good bone regeneration is possible even in a place where there is originally no bone, as demonstrated in a reconstruction model for a cleft jaw part in Examples described later.
In the present invention, by using the gelatin-containing granules, that is, by using gelatin in the form of granules, even in a site where pressure is normally applied to a bone defect such as a cleft jaw part, it becomes possible to retain the morphology.

<Gelatin>

The gelatin may be either naturally derived gelatin or recombinant gelatin, and the recombinant gelatin is preferable.

The recombinant gelatin means a polypeptide or protein-like substance having a gelatin-like amino acid sequence produced by genetic recombination technology.

The gelatin used in the present invention preferably has repetitions of a sequence represented by Gly-X-Y, which is characteristic of collagen (in the formula, X and Y each independently represent any one of amino acid). Here, the plurality of Gly-X-Y's may be the same as or different from each other. Preferably, the cell adhesion signal is contained in two or more sequences in one molecule. As the gelatin used in the present invention, gelatin having an amino acid sequence derived from a partial amino acid sequence of collagen can be used. For example, gelatin described in EP1014176B, US6992172B, WO2004/85473A, WO2008/103041A, and the like can be used, and are not limited thereto. The gelatin used in the present invention is preferably gelatin of the following aspects.

The recombinant gelatin has excellent bioaffinity due to the original performance of natural gelatin, has no concern about bovine spongiform encephalopathy (BSE) due to non-natural origin, and is excellent in non-infectivity. In addition, since recombinant gelatin is more uniform than natural gelatin and a sequence thereof is determined, it is possible to precisely design gelatin with less blurring due to crosslinking or the like in terms of strength and decomposability.

A molecular weight of the gelatin is not particularly limited, and is preferably 2000 or more and 100000 or less (2 kDa or more and 100 kDa or less), more preferably 2500 or more and 95000 or less (2.5 kDa or more and 95 kDa or less), still more preferably 5000 or more and 90000 or less (5 kDa or more and 90 kDa or less), and most preferably 10000 or more and 90000 or less (10 kDa or more and 90 kDa or less).

The molecular weight distribution of the gelatin is not particularly limited, and an area of the maximum molecular weight peak in the molecular weight distribution measurement contains gelatin which is preferably 70% or more, more preferably 90% or more, and most preferably 95% or more, of a total area of all the molecular weight peaks. A molecular weight distribution of the gelatin can be measured by a method described in WO2017/170342A.

The gelatin preferably has repetitions of a sequence represented by Gly-X-Y, which is characteristic of collagen. Here, the plurality of Gly-X-Y's may be the same as or different from each other. In Gly-X-Y, Gly represents glycine, and X and Y represent a predetermined amino acid (preferably a predetermined amino acid other than glycine). The sequence represented by Gly-X-Y, which is characteristic of collagen, is a very specific partial structure in amino acid composition and sequence of gelatin or collagen as compared with other proteins. In this part, glycine accounts for about one-third of the whole, and is repeated once in three in the amino acid sequence. The glycine is the simplest amino acid, has less binding to an arrangement of molecular chains, and contributes significantly to the regeneration of a helix structure during gelation. The amino acids represented by X and Y contain a large amount of imino acids (proline or oxyproline), and preferably account for 10% to 45% of the whole. Preferably 80% or more, still more preferably 95% or more, and most preferably 99% or more of the amino acids in the gelatin sequence have a repeating structure of Gly-X-Y.

In the common gelatin, charged polar amino acids and uncharged polar amino acids are present in a 1:1 ratio. Here, the polar amino acids specifically refer to cysteine, aspartic acid, glutamic acid, histidine, lysine, asparagine, glutamine, serine, threonine, tyrosine, and arginine. Among these, the polar uncharged amino acids are cysteine, asparagine, glutamine, serine, threonine, and tyrosine. In the gelatin used in the present invention, the ratio of polar amino acids to all the constituent amino acids is 10% to 40%, and preferably 20 to 30%. Moreover, the ratio of the uncharged amino acids in the polar amino acids is preferably 5% or more and less than 20%, and preferably 5% or more and less than 10%. Furthermore, the sequence does not contain preferably any one amino acid, and preferably two or more amino acids of serine, threonine, asparagine, tyrosine, and cysteine.

In general, the smallest amino acid sequence that acts as a cell adhesion signal in a polypeptide is known (for example, "Pathological Physiology" Vol. 9, No. 7 (1990), p. 527 published by Nagai Publishing Co., Ltd.). The gelatin used in the present invention preferably has two or more of these cell adhesion signals in one molecule. Specific sequences preferably include an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and a HAV sequence, which are represented by single-letter amino acid notation, in that there are many types of cells to adhere. More preferably, an RGD sequence, a YIGSR sequence, a PDSGR sequence, an LGTIPG sequence, an IKVAV sequence, and a HAV sequence are mentioned, and particularly preferably an RGD sequence is mentioned. Among the RGD sequences, the ERGD sequence is preferable. By using the gelatin having the cell adhesion signal, the amount of substrate produced by cells can be improved. For example, in a case of cartilage differentiation using mesenchymal stem cells as cells, the production of glycosaminoglycan (GAG) can be improved.

Regarding the arrangement of the RGD sequence in the gelatin used in the present invention, the number of amino acids between RGDs is preferably not uniform between 0 and 100, and preferably between 25 and 60.

A content of the minimum amino acid sequence is preferably 3 to 50, more preferably 4 to 30, and particularly preferably 5 to 20 per protein molecule from the viewpoint of cell adhesion and proliferation. The content thereof is most preferably 12.

In the gelatin used in the present invention, a ratio of RGD motif to a total number of amino acids is preferably at least 0.4%. In a case where the gelatin contains 350 or more amino acids, it is preferable that each stretch of 350 amino acids contains at least one RGD motif. The ratio of the RGD motif to the total number of amino acids is more preferably at least 0.6%, still more preferably at least 0.8%, even more preferably at least 1.0%, still further preferably at least 1.2%, and most preferably at least 1.5%. The number of the RGD motifs in the peptide is preferably at least 4, more preferably at least 6, still more preferably at least 8, and even more preferably 12 or more and 16 or less, per 250 amino acids. The ratio 0.4% of the RGD motif corresponds to at least one RGD sequence per 250 amino acids. Since the number of the RGD motifs is an integer, gelatin consisting of 251 amino acids should contain at least two RGD sequences to satisfy the feature of at least 0.4%. The gelatin preferably contains at least 2 RGD sequences per 250 amino acids, more preferably contains at least 3 RGD sequences per 250 amino acids, and still more preferably contains at least 4 RGD sequences per 250 amino acids. Further embodiments of the gelatin in the present invention include at least 4 RGD motifs, preferably at least 6, more preferably at least 8, and still more preferably 12 or more and 16 or less of RGD motifs.

The gelatin may be partially hydrolyzed.

The gelatin used in the present invention is preferably represented by A-[(Gly-X-Y)$_n$]$_m$-B. n pieces of X's each independently represent one of the amino acids, and n pieces of Y's each independently represent one of the amino acids. m preferably represents an integer of 2 to 10, and more preferably an integer of 3 to 5. n is preferably an integer of 3 to 100, more preferably an integer of 15 to 70, and most preferably an integer of 50 to 65. A represents a predetermined amino acid or amino acid sequence, and B represents a predetermined amino acid or amino acid sequence. The n pieces of Gly-X-Y's may be the same as or different from each other.

The gelatin used in the present invention more preferably has Formula: Gly-Ala-Pro-[(Gly-X-Y)$_{63}$]$_3$-Gly (in the Formula, 63 X's each independently represent one of the amino acids, 63 Ys each independently represent one of the amino acids, and 63 Gly-X-Y's may be the same as or different from each other).

It is preferable to bind a plurality of naturally occurring collagen sequence units to the repeating unit. The naturally occurring collagen referred to here may be any naturally occurring collagen, and is preferably type I, type II, type III, type IV, or type V collagen. The collagen is more preferably, type I, type II, or type III collagen. According to another embodiment, the origin of the collagen is preferably human, bovine, porcine, mouse, or rat, and more preferably human.

An isoelectric point of the gelatin used in the present invention is preferably 5 to 10, more preferably 6 to 10, and still more preferably 7 to 9.5. The measurement of the isoelectric point of the gelatin can be performed by measuring the pH after passing a 1% by mass gelatin solution through a mixed crystal column of cation and anion exchange resins, according to isoelectric focusing method (see Maxey, C. R. (1976; Phitogr. Gelatin 2, Editor Cox, P. J. Academic, London, Engl.).

The gelatin is preferably not deaminated.

The gelatin is preferably telopeptide-free.

The gelatin is preferably a substantially pure polypeptide prepared with a nucleic acid encoding an amino acid sequence.

The gelatin used in the present invention is particularly preferably any one of:

(1) a peptide consisting of amino acid sequence represented by SEQ ID NO: 1;
(2) a peptide consisting of an amino acid sequence in which one or several amino acids are deleted, substituted, or added in the amino acid sequence represented by SEQ ID NO: 1 and having bioaffinity; or
(3) a peptide consisting of an amino acid sequence having 80% or more (more preferably 90% or more, particularly preferably 95% or more, and most preferably 98% or more) of sequence identity with the amino acid sequence represented by SEQ ID NO: 1, and having bioaffinity.

The " 1 or several" in the "amino acid sequence in which one or several amino acids are deleted, substituted, or added" preferably means 1 to 20, more preferably 1 to 10, still more preferably 1 to 5, and particularly preferably 1 to 3.

A hydrophilicity value "1/IOB" value of the gelatin used in the present invention is preferably 0 to 1.0. The value is more preferably 0 to 0.6, and still more preferably 0 to 0.4. The IOB is a hydropathicity index based on an organic conceptual diagram showing the polarity/non-polarity of an organic compound proposed by Akira Fujita, and details thereof are described in, for example, "Pharmaceutical Bulletin", vol. 2, 2, pp. 163-173 (1954), "Fields of Chemistry" vol. 11, 10, pp. 719-725 (1957), "Fragrance Journal", vol. 50, pp. 79-82 (1981), and the like. In short, a source of all organic compounds is methane ($CH_4$), and all other compounds are regarded as derivatives of methane. A certain numerical value is set for each of a carbon number, a substituent, a transformation part, a ring, and the like, and scores thereof are added to determine an organic value (OV) and an inorganic value (IV). This value is plotted on a diagram with the organic value on an X-axis and the inorganic value on a Y-axis. The IOB in the organic conceptual diagram refers to a ratio of the inorganic value (IV) to the organic value (OV) in the organic conceptual diagram, that is, "inorganic value (IV)/organic value (OV)". For details on the organic conceptual diagram, refer to "New Edition Organic Conceptual Diagram -Basics and Applications-" (written by Yoshio Koda et al., Sankyo Publishing, 2008). In the present specification, the reciprocal of IOB, "1/IOB" value, represents the hydropathicity. The notation represents that the smaller the "1/IOB" value (closer to 0), the more the hydrophilic.

By setting the "1/IOB" value of the gelatin used in the present invention within the above range, the hydrophilicity is high and water absorption becomes high.

The gelatin used in the present invention preferably has hydropathicity index represented by Grand average of hydropathicity (GRAVY) value of preferably 0.3 or less and -9.0 or more, and more preferably 0.0 or less and -7.0 or more. The Grand average of hydropathicity (GRAVY) value can be obtained by methods described in "Gasteiger E., Hoogland C., Gattiker A., Duvaud S., Wilkins M. R., Appel R. D., Bairoch A.; Protein Identification and Analysis Tools on the ExPASy Server; (In) John M. Walker (ed): The Proteomics Protocols Handbook, Humana Press (2005). pp. 571-607" and "Gasteiger E., Gattiker A., Hoogland C., Ivanyi I., Appel R.D., Bairoch A.; ExPASy: the proteomics server for in-depth protein knowledge and analysis.; Nucleic Acids Res. 31: 3784-3788 (2003)".

By setting the "GRAVY" value of the gelatin used in the present invention within the above range, the hydrophilicity is high and water absorption becomes high.

The recombinant gelatin used in the present invention can be produced by a gene recombination technique known to those skilled in the art, and can be produced in accordance with the methods described in, for example, EP1014176A2, US6992172B, WO2004/85473A, WO2008/103041A, and the like. Specifically, a gene encoding an amino acid sequence of a predetermined recombinant gelatin is obtained, and is incorporated into an expression vector to prepare a recombinant expression vector, and this vector is introduced into an appropriate host to prepare a transformant. Since the recombinant gelatin is produced by culturing the obtained transformant in an appropriate culture medium, the gelatin used in the present invention can be prepared by collecting the recombinant gelatin produced from the culture.

<Gelatin-containing granules>

The gelatin-containing granules in the present invention mean granules in which all or a part of components thereof are gelatin. Examples of the gelatin-containing granules include granules consisting of only gelatin and granules obtained by coating an inorganic component with gelatin. Two or more types of gelatin-containing granules may be mixed and used. As the gelatin-containing granules, the granules consisting of only gelatin are preferable. In addition, the inorganic component refers to a component containing alkali metals of Group 1, alkaline earth metals of Group 2, transition metals of Groups 3 to 12, typical metals of Groups 13 to 15, and metalloid elements such as boron, silicon, and germanium. The inorganic component is preferably ceramics, titanium, stainless steel, hydroxyapatite, and β-tricalcium phosphate which have high bioaffinity.

Granules consisting of only gelatin can be produced, for example, by producing a gelatin porous body (gelatin block) by freeze-drying an aqueous solution containing gelatin, and pulverizing the gelatin porous body with a pulverizer.

The granules obtained by coating the inorganic component with gelatin can be produced, for example, by immersing the granules of the inorganic component in an aqueous solution of gelatin and coating the granules of the inorganic component with gelatin by air-drying or freeze-drying.

The gelatin in the gelatin-containing granules may be crosslinked or uncrosslinked, and crosslinked gelatin is preferable. As a common crosslinking method, thermal crosslinking, crosslinking with aldehydes (for example, formaldehyde, glutaraldehyde, and the like), crosslinking with a condensing agent (such as carbodiimide and cyanamide), enzymatic crosslinking, photocrosslinking, ultraviolet crosslinking, hydrophobic interaction, hydrogen bond, ionic interactions, and the like are known, and the above-mentioned crosslinking method can also be used in the present invention. The crosslinking method used in the present invention is more preferably thermal crosslinking, ultraviolet crosslinking, or enzymatic crosslinking, and particularly preferably thermal crosslinking.

In a case where crosslinking with an enzyme is performed, the enzyme is not particularly limited as long as it has a crosslinking action between polymer materials. The crosslinking can be performed by preferably using transglutaminase and laccase, and most preferably using transglutaminase. Specific examples of protein enzymatically crosslinking with the transglutaminase are not particularly limited as long as it is a protein having a lysine residue and a glutamine residue. The transglutaminase may be derived from a mammal or a microorganism. Specific examples thereof include ACTIVA series manufactured by Ajinomoto Co., Inc. and transglutaminase derived from mammals sold as a reagent, for example, guinea pig liver-derived transglutaminases manufactured by Oriental Yeast Co., Ltd., Upstate USA Inc., Biodesign International, and the like, goat-derived transglutaminase, rabbit-derived transglutaminase, and human-derived blood coagulation factor (Factor XIIIa, Haematologic Technologies, Inc.).

A reaction temperature when performing the crosslinking (for example, thermal crosslinking) is not particularly limited as long as the crosslinking is possible, and is preferably -100°C to 500°C, more preferably 0°C to 300°C, still more preferably 50°C to 300°C, even more preferably 100°C to 250°C, and still further preferably 120°C to 200°C.

A degree of crosslinking of the gelatin-containing granules in the present invention is not particularly limited, and is preferably 0.4 or more, more preferably 0.6 or more and 30 or less, still more preferably 0.8 or more and 15 or less, and particularly preferably 1.0 or more and 7 or less.

A method for measuring the degree of crosslinking (number of crosslinks per molecule) of gelatin-containing granules is not particularly limited, and the measurement can be performed by, for example, a TNBS (2,4,6-trinitrobenzenesulfonic acid) method. Specifically, a sample obtained by mixing the gelatin-containing granules, a $NaHCO_3$ aqueous solution, and a TNBS aqueous solution and reacting at 37°C for 3 hours and then stopping the reaction and a blank obtained by mixing gelatin-containing granules, a $NaHCO_3$ aqueous solution, and a TNBS aqueous solution, and immediately after this, and stopping the reaction are prepared respectively, and the absorbance (345 nm) of the sample and the blank diluted with pure water were measured. The degree of crosslinking (number of crosslinks per molecule) can be calculated from the following Formula 2 and Formula 3.

$$(\text{Formula 2}) \quad (As\text{-}Ab)/14600 \times V/w$$

(Formula 2) shows the amount of lysine (molar equivalent) per 1 g of gelatin-containing granules.

(In the formula, As represents a sample absorbance, Ab represnets the blank absorbance, V is a reaction solution volume (g), and w is the gelatin-containing granule mass (mg)).

$$(\text{Formula 3}) \quad 1 - (\text{Sample (Formula 2)}/\text{Uncrosslinked polymer (Formula 2)}) \times 34$$

(Formula 3) shows the number of crosslinks per molecule.

A size of each granule of the gelatin-containing granules is not particularly limited, and is preferably 100 $\mu$m or more and 2000 $\mu$m or less, more preferably 200 $\mu$m or more and 1800 $\mu$m or less, still more preferably 300 $\mu$m or more and 1700 $\mu$m or less, and particularly preferably 500 $\mu$m or more and 1500 $\mu$m or less. By setting the size of each granule of the gelatin-containing granules within the above range, a good bone regeneration effect can be exhibited.

The size of one granule can be defined by a size of the sieve used to separate the granules. For example, the granules remaining on a sieve in a case where the granules passed through the sieve of 2000 $\mu$m are sieved to the sieve of 500 $\mu$m can be granules having a size of 500 to 2000 $\mu$m.

<Stem cell>

The stem cells are used in the present invention.

The stem cells are cells that have an ability to proliferate by themselves (self-renewal ability) and an ability to differentiate into a specific cell (multipotency).

As the stem cells, for example, mesenchymal stem cells (MSC), induced pluripotent stem (iPS) cells, embryonic stem (ES) cells, germline stem (GS) cells, hematopoietic stem cells, amnion cells, umbilical cord blood cells, adipose-derived stem cells, bone marrow-derived stem cells, dental pulp-derived stem cells, myocardial stem cells, synovial-derived stem cells, nerve stem cells, and the like can be used. As the stem cells, mesenchymal stem cells (MSC), induced pluripotent stem (iPS) cells, or embryonic stem (ES) cells are preferable, and adipose-derived stem cells, bone marrow-derived stem cells, or dental pulp-derived stem cells are still more preferable.

The stem cells to be used may be one type or a combination of a plurality of types of cells. The cells to be used are preferably animal cells, more preferably vertebrate-derived cells, and particularly preferably human-derived cells. Moreover, the origin of the cell may be any of an autologous cell or an allogeneic cell. The stem cells to be used may include cells other than stem cells.

The number of the stem cells is preferably $1.0 \times 10^3$ or more, more preferably $2.5 \times 10^3$ or more, still more preferably $1.0 \times 10^4$ or more, and even more preferably $2.5 \times 10^4$ or more, per 1 mg of gelatin-containing granules. An upper limit is not particularly limited, and generally $1.0 \times 10^6$ or less and may be $1.0 \times 10^5$ or less.

The number of the stem cells is preferably $2.0 \times 10^2$ or more, more preferably $5.0 \times 10^2$ or more, still more preferably $2.0 \times 10^3$ or more, and even more preferably $5.0 \times 10^3$ or more, per gelatin-containing granule. An upper limit is not particularly limited, and generally $2.0 \times 10^5$ or less and may be $2.0 \times 10^4$ or less.

<Bone regenerative agent>

The bone regenerative agent according to the embodiment of the present invention can be produced by mixing the gelatin-containing granules and the stem cells together. For example, the gelatin-containing granules are infiltrated with a cell suspension containing stem cells on a plate such as a 96-well plate and cultured for a predetermined time. Then, the gelatin-containing granules infiltrated with the cells are placed in a culture container containing an appropriate culture medium and vibrated. Accordingly, the bone regenerative agent according to the embodiment of the present invention can be produced.

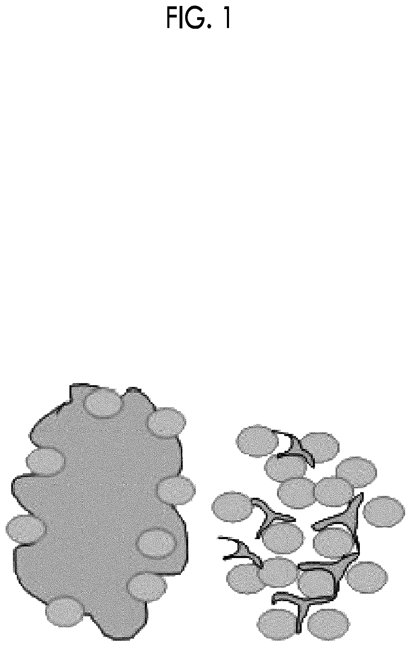

Fig. 1 is an image of gelatin-containing granules + stem cells (left) and petal-shaped blocks + stem cells (right). The gelatin-containing granules + stem cells (left) is an image of the bone regenerative agent according to the embodiment of the present invention.

As can be seen from Fig. 1, the number of cells shown in an oval shape is smaller in a case where gelatin-containing granules are used.

Specifically, in the case of gelatin-containing granules + stem cells, as described above, the number of cells per 1 mg of gelatin-containing granules is preferably $1.0 \times 10^3$ or more, and is generally $2.0. \times 10^5$ or less. On the other hand, in the case of the petal-shaped block + stem cell, for example, in Patent Document 2, $1.0 \times 10^6$ per 1 mg of petal-shaped block (= 0.001 $\mu$g block per cell). There is a difference of tens to thousands of times in the number of cells per granule or block.

In the bone regenerative agent comprising the gelatin-containing granules and the stem cells according to the embodiment of the present invention, it is preferable that the stem cells are attached to the surface of the gelatin-containing granules. Specifically, it is preferable that 70% or more (preferably 80% or more, and more preferably 90% or more) of the stem cells are attached to the surface or the pores on the surface of the gelatin-containing granules. It can be confirmed and measured by photographs that the stem cells are attached to the surface or the pores on the surface of the gelatin-containing granules. Specifically, by preparing frozen sections of the bone regenerative agent and then staining with hematoxylin/eosin (HE) staining, it can be confirmed that 70% or more of the observable stem cells are attached to the surface or pores on the surface of the gelatin-containing granules. As described above, in a case where the bone defect site in the same space is filled with the bone regenerative agent by attaching the stem cells to the surface or the pores on the surface of the gelatin-containing granules, there is an advantage that the number of stem cells to be used can be small.

The bone regenerative agent according to the embodiment of the present invention can promote and induce bone regeneration by being administered to a subject (for example, a mammal such as a human) who needs bone regeneration. Preferably, the bone regenerative agent according to the embodiment of the present invention can be directly administered to a bone-deficient site in a living body.

The dose, usage, and dosage form of the bone regenerative agent according to the embodiment of the present invention can be appropriately determined according to the purpose of use. For example, the bone regenerative agent according to the embodiment of the present invention may be directly administered to a target site in a living body, or may be suspended in a liquid excipient such as an aqueous solvent such as distilled water for injection, physiological saline for injection, and a buffer solution having a pH of 5 to 8 (phosphate-based, citric acid-based, and the like) and administered by, for example, injection or application. In addition, the bone regenerative agent may also be mixed with an appropriate excipient to form an ointment, a gel, a cream, or the like and then applied.

Preparing of the bone regenerative agent according to the embodiment of the present invention can be performed according to a method known to those skilled in the art. For example, in a case where the preparation carrier is a liquid, the preparation can be dissolved or dispersed, and in a case where the preparation carrier is a powder, the preparation can be mixed or adsorbed. Furthermore, as needed, pharmaceutically acceptable additives (for example, a preservative, a stabilizer, an antioxidant, an excipient, a binder, a disintegrant, a wetting agent, a lubricant, a coloring agent, a fragrance, a flavoring agent, a coating, a suspending agent, an emulsifier, a solubilizing agent, a buffer, a tonicity agent, a plastic agent, a surfactant, a soothing agent, and the like) can also be contained.

The dose of the gelatin is not particularly limited, and is, for example, 1 to 100 mg, and preferably 1 to 50 mg per 1 $cm^2$ of the surface area of the living body to be administered.

Examples of a target of the bone regenerative agent according to the embodiment of the present invention include bone defects and fractures due to trauma or the like, oral surgery diseases and treatments thereof (such as alveolar bone defect, cleft lip and palate, cleft jaw, periodontal disease, and periodontal disease bone defect), osteoporosis, arthropathy, and spinal fusion (for spondylolysis, spondylolisthesis, and vertebral fractures). The bone regenerative agent according to the embodiment of the present invention can be particularly preferably used for the cleft jaw part.

The present invention will be described in more detail with reference to the following Examples, but the present invention is not limited to Examples.

Examples

[Preparation of gelatin-containing granules]

<1> Recombinant peptide (recombinant gelatin)

The following CBE3 was prepared as a recombinant peptide (recombinant gelatin) (described in WO2008/103041A).

CBE3:

Molecular weight: 51.6 kD
Structure: GAP [(GXY)$_{63}$]$_3$G
Number of amino acids: 571
RGD sequence: 12 sequences
Imino acid content: 33%

Almost 100% of the amino acids have a repeating structure of GXY. The amino acid sequence of CBE3 does not include serine, threonine, asparagine, tyrosine, and cysteine. The CBE3 has an ERGD sequence.

Isoelectric point: 9.34
GRAVY value: -0.682
1/IOB value: 0.323

Amino acid sequence (SEQ ID NO: 1 in the sequence listing) (Same as SEQ ID NO: 3 in WO2008/103041A, but X at the end is corrected to "P")

GAP(GAPGLQGAPGLQGMPGERGAAGLPGPKGERGDAGPKGADGAPGAPGL

QGMPGERGAAGLPGPKGERGDAGPKGADGAPGKDGVRGLAGPIGPPGERGAAGLPG

PKGERGDAGPKGADGAPGKDGVRGLAGPIGPPGPAGAPGAPGLQGMPGERGAAGLP

GPKGERGDAGPKGADGAPGKDGVRGLAGPP)$_3$G

<2> Preparation of granules of recombinant gelatin porous body

An aluminum cylindrical cup-shaped container having a thickness of 5 mm and a diameter of 98 mm was prepared. In a cylindrical cup, in a case where a curved surface is a side surface, the side surface is closed with 1 mm aluminum, and a bottom surface (circular shape of a flat plate) is also closed with 5 mm aluminum. On the other hand, an upper surface has an open shape. Also, Teflon (registered trademark) having a wall thickness of 3 mm is uniformly spread only on an inside of the side surface, and as a result, an inner diameter of the cylindrical cup is 90 mm. Hereinafter, this container will be referred to as a cylindrical container.
A gelatin aqueous solution containing 7.5% by mass of the recombinant gelatin was poured into a cylindrical container in an amount of about 18 ml, and allowed to stand in a freezer at -50°C (Hitachi, ultra-low temperature freezer RS-U30T) for 1 hour or longer to obtain a frozen gelatin block. This gelatin block was freeze-dried (Takara, TF5-85ATNNN), pulverized with a pulverizer (Quadro, Komil U10) on a screen having a pore size of about 1 mm, and a fraction under a sieve having an opening of 1.4 mm was collected. The obtained gelatin-containing granules were treated in a nitrogen atmosphere at 135°C (Yamato Kagaku, DP-43) for 4.75 hours to obtain granules of gelatin porous body (hereinafter, also referred to as gelatin granules). A degree of crosslinking of the granules by thermal crosslinking was 1.4.

[Preparation of dental pulp-derived stem cells]

At the outpatient department of Oral Surgery, Aichi Gakuin University School of Dentistry, a patient and guarantor of the patient signed a consent form for the provision and research use of the extracted deciduous tooth and pulp, and then the pulp was removed from the extracted tooth.
An oral cavity was disinfected before tooth extraction, and extraction of the right maxillary milk incisor (5-year-old child) was performed. Immediately after extracted, the extracted tooth was placed in a 15 ml centrifuge tube containing 10 ml of MEMα (Wako) (20% FBS (gibco) + 1% Penicillin-Streptomycin (Wako) (hereinafter P/S)), then stored at 4°C in a cooler box, and carried from the university hospital to the oral anatomy laboratory on the Kusumoto campus of the Faculty of Dentistry, Aichi Gakuin University. The cells were stored in a refrigerator (below 4°C) until a treatment. The MEMα means Minimum Essential Medium α, and FBS means Fetal Bovine Serum.
Isolation of cells from the dental pulp was performed in a clean bench in the oral anatomy laboratory. Specifically, the dental pulp was removed from the crown and root of the deciduous tooth using a dental K file (sales company: Manny, brand name: Manny K file, size 15, tip diameter 0.15 mm) from extracted deciduous tooth (roots were absorbed and the medullary cavity was open, so these were not divided). The removed dental pulp was put into a 15 ml centrifuge tube prepared in advance with 4 ml of D-PBS (Wako) (FBS 2% P/S 1%) and stirred to wash away blood and the like. Next, after the pulp had settled, only a supernatant was removed with a pipette. This operation was performed 5 times.

D-PBS represents Dulbeccoline phosphate buffered saline.

After washing, cells are isolated from the dental pulp by an enzymatic treatment. 3 ml of washed dental pulp and cell detachment enzyme Accutase (Innovative Cell Technologies) were added to a 15 ml centrifuge tube, and stirred for 30 minutes at a frequency of 118 rpm at 37°C in a Bio Shaker (TAITEC Bio Shaker V BR-36:). After stirring for 30 minutes, PRIME-XV (registered trademark) MCS XSFM 7 ml (under 37°C environment) (Irvine Scientific) was added to 3 ml of Accutase, and pipetting was performed 20 times while stopping the enzymatic reaction to perform operation of isolating cells from the dental pulp. After pipetting, centrifugation was performed with a centrifuge (100G, 5 minutes) and then only the supernatant solution was removed. 5.7 ml of PRIME-XV (registered trademark) MCS XSFM (P/S 1% (Irvine Scientific)) was added, and pipetting was performed. The cells were seeded by adding 1.9 ml of each cell suspension to 3-well of 6-well-plate (coated with 2.5 $\mu$g/ml of PRIME-XV (registered trademark) Human Fibronectin (Irvine Scientific) for 12 hours). For one week after seeding, a culture medium was left unchanged and the cells were allowed to entransplantation in the dish.

One week later, after confirming cell entransplantationment and subsequent proliferation, in a case where the cells became 80% confluent, the cells were collected from the dish and seeded again in 6-well-plate (coated with 2.5 $\mu$g / ml of PRIME-XV® Human Fibronectin (Irvine Scientific) for 12 hours) with $1.0 \times 10^4$ cells, and 3 ml (containing 1% P/S) of culture medium PRIME-XV (registered trademark) MCS XSFM culture medium was added. It was determined to use the cell group as a first subculture, and cells of a third subculture were set to be used for this experiment. The cells obtained above are cells containing dental pulp-derived stem cells.

[Preparation of bone regenerative agent comprising gelatin-containing granules and stem cells]

Cells containing deciduous dental pulp-derived stem cells (hereinafter, DDPSCs) cultured up to the third subculture were collected, and cell suspensions having $1.0 \times 10^5$ cells/18 $\mu$l, $5.0 \times 10^4$ cells/18 $\mu$l, and $1.0 \times 10^4$ cells/18 $\mu$l were prepared. 3 mg of a thermally crosslinked substance Fuji Bone Graft (gelatin granules) treated at 135°C for 4.75 hours in a nitrogen atmosphere was prepared, a previously prepared cell suspension was infiltrated on a 96-well plate, and was cultured for 10 minutes in a carbon dioxide incubator (SANYO, MCO-18AIC (UV)) ($CO_2$: 5% in a 37°C environment) in an environment at 37°C. Thereafter, 10 ml of PRIME-XV (registered trademark) MCS XSFM (P/S 1%) and 3 mg of the gelatin granules infiltrated in advance were added to 15 ml of Cell reactor (Greiner bio-one), and the mixture was vibrated for 24 hours (frequency 118 rpm/min) in Bio shaker (under 37°C environment) to prepare a transplant (gelatin granules + cells).

The average number of granules per 3 mg of granules was 13 (n = 3). In the case of $1 \times 10^5$ cells, there are about $7.7 \times 10^3$ cells per granule.

In order to measure a colonization rate of dental pulp-derived stem cells in the gelatin granules, after infiltrating the cells into the gelatin granules in advance, the gelatin granules were moved from the 96-well-plate to determine the number of cells remaining on the bottom surface and the culture medium. Next, after giving Bio Shaker for 24 hours, the gelatin granules in the Cell reactor were moved, and the cells remaining in the culture medium of PRIME-XV (registered trademark) MCS XSFM were collected and the number of cells was counted. From these measured values, the number of cells that could not be colonized in gelatin granules was calculated, and the cell colonization rate was examined.

From the above examination, results in which the colonization rate of cells in gelatin granules was 85.5% in a case where $1.0 \times 10^5$ cells were seeded, 85.0% in a case where $5.0 \times 10^4$ cells were seeded, and 83.3% in a case where $1.0 \times 10^4$ cells were seeded could be obtained.

In addition, in a case where a section of gelatin granules on which cells have been colonized was prepared, it can be confirmed that by staining with hematoxylin/eosin (HE) staining, 70% or more of the dental pulp-derived stem cells that can be confirmed in the stained image attached to the surface or the pores on the surface of the gelatin-containing granules.

[Evaluation in rat parietal bone defect model] (See Example 1 of WO11/027850A)

As animals for transplantation, 17 nude rats (F344/NJcL-rnu/rnu nude rat, male, 10 weeks old, 0.3-0.5 kg) were used. For anesthesia of rats, a simple inhalation anesthesia device NARCOBIT-E (type 2) for experiments on small animals such as mice and rats was used, and isoflurane inhalation anesthetic solution (Phaiser) was used as an anesthetic, and anesthesia was performed by inhalation anesthesia (concentration was 4.5% at introduction and 3.5% during maintenance). The rat skull skin and periosteum were incised with a No. 15 scalpel and exfoliated to expose the skull. In order to prepare a constant bone defect, using a trephine bar $\varphi$4.0 mm/5.0 mm (Hanger & Meisinger), a circular bone defect with a diameter of 5 mm was created so as to be located in the center of the left side of the parietal bone, avoiding the central suture of the skull.

Next, a sample for transplantation to the prepared bone defect part was prepared. 3 mg of gelatin granules containing DDPSCs ($1.0 \times 10^5$ cells/18 $\mu$l, n = 6) as a first group, 3 mg of gelatin granules containing DDPSCs (5.0 $\times$

$10^4$ cells/18 μl, n = 6) as a second group, and 3 mg of gelatin granules containing DDPSCs (1.0 × $10^4$ cells/18 μl, n = 5) as a third group were transplanted to the bone defect part. After transplanting, the periosteum was returned to an original position and the skin was sutured with silk thread.

After transplanting, imaging using 3D micro X-ray CT for small laboratory animals CosmoScan GX (Rigaku) (tube voltage: 90 kV, tube current: 88 μA FOV [mm]: φ30 × H30, shooting time: 18 seconds, voxel size [μm]: 90 × 90 × 90) was performed every week until the 4th week after transplanting, and the bone formation amount was compared using software of by 4viewer 2011 (kitasennju Radist Dental Clinic I-View Image Center, Tokyo, Japan) based on the image.

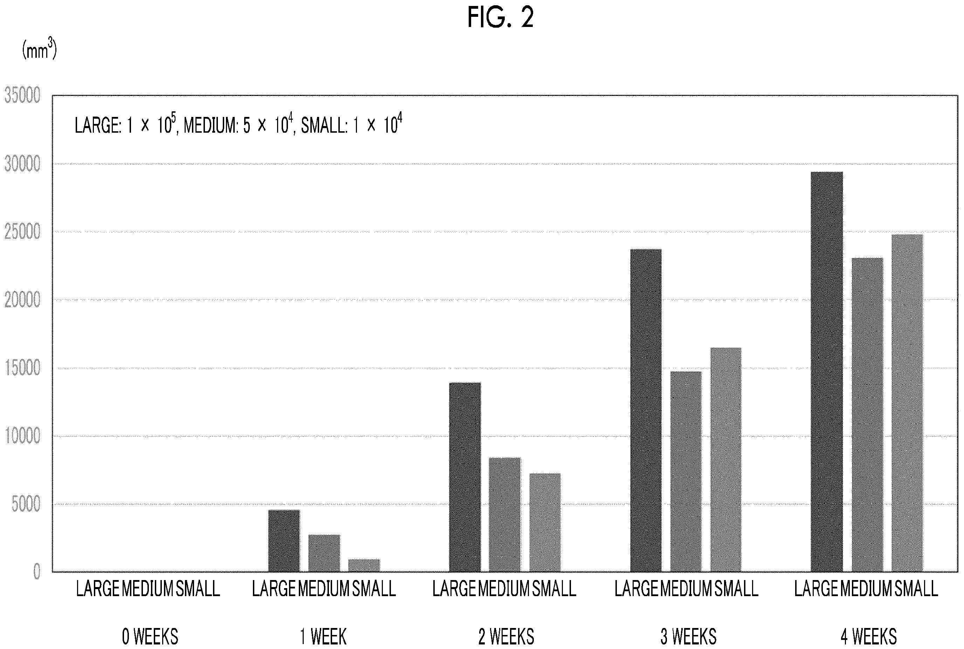

Table 1 and Fig. 2 show measurement results of changes in bone volume over time in each transplantation group.

From the results shown in Table 1 and Fig. 2, an increase in bone formation amount in each transplantation group was observed. In addition, in the DDPCs 1 × $10^5$ cells transplanted rat, a significantly higher value was observed in the bone formation amount one week after the transplantation than in the DDPCs 1 × $10^4$ cells transplanted rat (t value = 0.038).

In addition, significant difference was not observed in the bone formation amount one week after the transplantation between the DDPCs 1 × $10^5$ cells transplanted rat and the DDPCs 5 × $10^4$ cells transplanted rat (t value = 0.173). After the second week, a difference in the bone formation amount was observed, but a significant difference was not observed.

[Table 1]

| Bone volume of gelatin granules + dental pulp-derived stem cell transplantation group in each period (mm3) | | | |
|---|---|---|---|
| | 1 × $10^5$ cells per nude rat | 5 × $10^4$ cells per nude rat | 1 × $10^4$ cells per nude rat |
| 0 Weeks | 0 | 0 | 0 |
| 1 Week | 4565.5 | 2749 | 929.4 |
| 2 Weeks | 13912.83333 | 8432.333333 | 7269.4 |
| 3 Weeks | 23677.33333 | 14759.16667 | 16488.6 |
| 4 Weeks | 29372 | 23045 | 24771.2 |

[Evaluation in cleft jaw part reconstruction model]

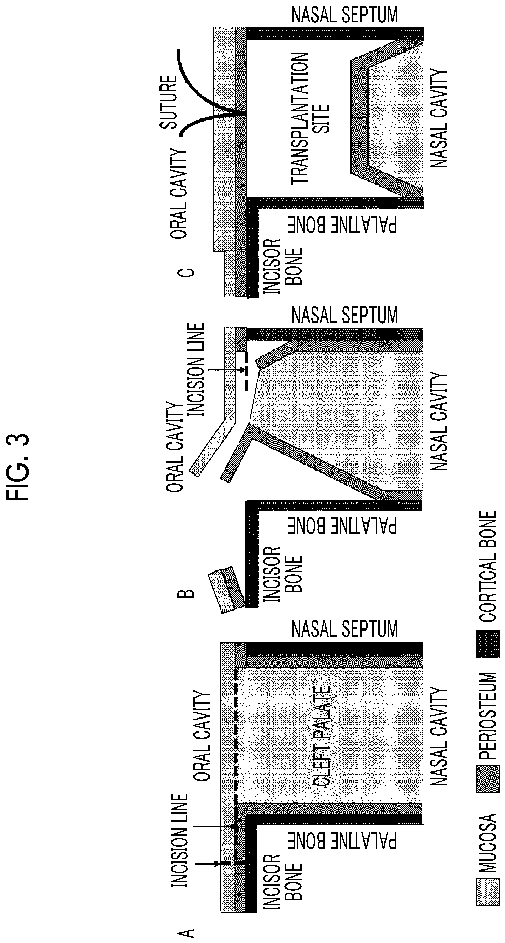

Triple mixed anesthesia 0.5 ml/100 g (0.3 mg/kg DOMITOR (registered trademark) (Nippon Zenyaku Kogyo Co., Ltd, Japan), 4.0 mg/kg of midazolam (SANDOZ, Japan), 5.0 mg/kg of Vetorphale (registered trademark) (Meiji Seika Pharma Co., Ltd, Japan), and 2.9 ml/kg of Otsuka Normal Saline (Otsuka Pharmaceutical Co., Ltd, Japan) were used to perform intraperitoneal anesthesia. Next, a cavity for transplanting the transplant is created (Fig. 3). First, silk thread is applied to the buccal mucosa on the side where the transplantation part is to be created, and the buccal mucosa is stretched with a mosquito to secure the field of view of the surgical field that will be the palate. No. 15 scalpel is used for incision of the palate mucosa of the palate. The incision is made from the incisor toward the first molar, and an incision with a depth of 10 mm is made on the incisor bone body to the subperiosteal bone (A in Fig. 3). In a case where the mucosal periosteal flap is peeled off from the incisor bone, the surface of the cortical bone of the incisor bone body can be observed. Bone cannot be observed unless the periosteum is peeled off. In a case where the mucosal periosteum is continuously peeled from the incisor bone side along the palatine bone toward the nasal cavity along the periosteum of the incision part, an invaginated cleft palate can be observed. The transplant will be transplanted into the invaginated cleft palate part. Next, the periosteum on the septal side is directed toward the nasal cavity and the peeling is continued. Next, the mucosa of the palate that covered the cleft palate is separated into the mucosa and the periosteum (B in Fig. 3). The peeled periosteum on the incisor bone/palatine bone side and the periosteum on the nasal septum side are pushed into the nasal cavity side using a sterile cotton ball (C in Fig. 3). The depth of the cleft palate part is measured with a measure, and in a case where a depth of about 5 mm is obtained from the oral mucosa, the invagination of the periosteum toward the nasal cavity is stopped. Since the site for transplanting the implant body can be created here, the transplant is transplanted here.

Two groups of the transplant created above were prepared. The first group was a group in which only 2 mg of gelatin granules were transplanted, and in this case, SD rats (male, 11 weeks old, 5 animals) were used. In a case of transplanting the transplant, bleeding from the cleft palate part of the rat was used, and the transplant was transplanted after being acclimated with blood. This group was designated as the control group. The second group is a group in which 1 × $10^5$ deciduous pulp-derived mesenchymal stem cells were seeded in 2 mg of gelatin granules. Immunodeficient nude rats (male, 11 weeks old, 5) were used for transplanting. For the preparation of a cell mixture, gelatin granules (2 mg) were seeded with $1 \times 10^5$ deciduous pulp-derived mesenchymal stem cells the day before.

After completion of each transplanting in both groups, a hypotonic incision was made in the periosteum on the incisor bone body side (outer side), it was confirmed that the upper surface of the rat cleft palate was covered with periosteum, and the mucosal periosteal flap was sutured with 4-0 silk thread to close the wound. This suture allowed the transplantation to remain at the transplantation site. After the transplanting surgery was completed, powdered food was given.

The degree of bone regeneration after transplantation was evaluated by micro-CT imaging before transplantation and 4 weeks after transplantation. The imaging condition was set to The exposure parameters: 18s, 90kV, and 88μA, voxel size: 45 μm, CT analysis software: 3 by 4 viewer 2011 (Kitasenjyu Radist Dental Clinic I-View Image Center, Tokyo, Japan), ROI size: $1.8 \times 2.7 \times 0.9$ mm.

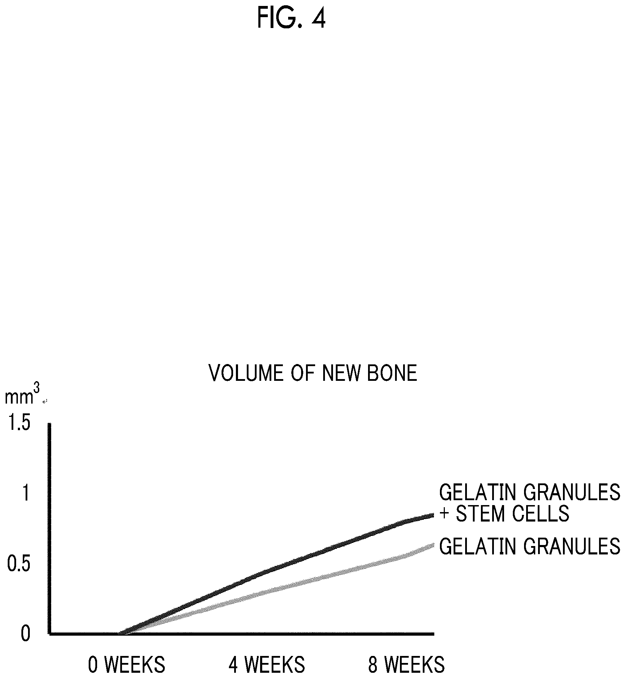

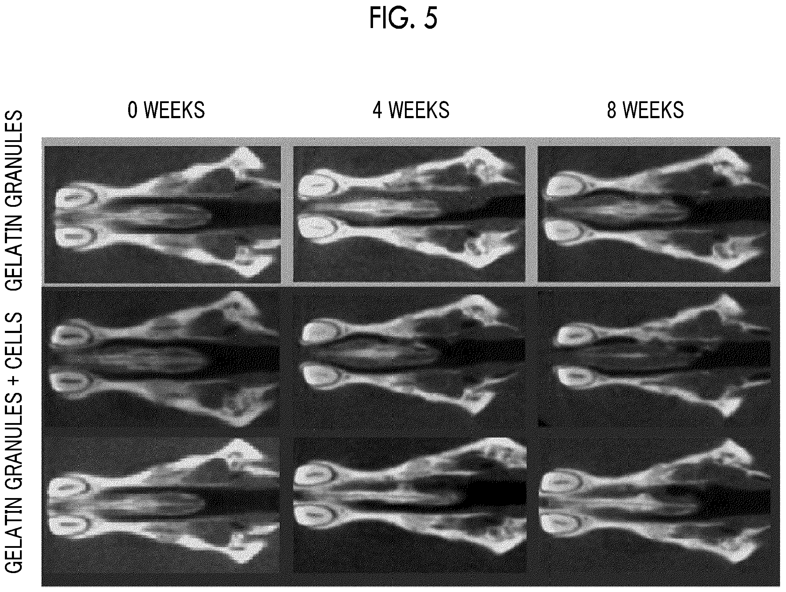

Table 2 and Fig. 4 show the results of comparing the volume of new bone between the two groups by CT analysis. Fig. 5 shows CT images (typical examples) of an affected area in each transplantation group.

An increase in volume of new bone was observed in the group to which gelatin granules + deciduous pulp-derived mesenchymal stem cells were administered and the group to which gelatin granules were administered. In addition, at 4 and 8 weeks after transplantation, the volume of new bone in the group of gelatin granules + mesenchymal stem cells derived from deciduous tooth pulp was significantly higher than that of the group in which only gelatin granules were transplanted.

[Table 2]

| Bone volume of gelatin granules transplantation group in each period (mm3) | | | | | | |
|---|---|---|---|---|---|---|
| | Object 1 | Object 2 | Object 3 | Object 4 | Object 5 | Average |
| 4 Weeks | 0.15066 | 0.434484 | 0.395604 | 0.223074 | 0.278001 | 0.2963646 |
| 8 Weeks | 0.487458 | 0.591948 | 0.479682 | 0.592434 | 0.71331 | 0.5729664 |

| Bone volume of gelatin granules + dental pulp-derived stem cell transplantation group in each period (mm3) | | | | | | |
|---|---|---|---|---|---|---|
| | Object 1 | Object 2 | Object 3 | Object 4 | Object 5 | Average |
| 4 Weeks | 0.332424 | 0.479682 | 0.58563 | 0.486486 | 0.302778 | 0.4374 |
| 8 Weeks | 0.603126 | 0.79947 | 1.025946 | 0.885006 | 0.693036 | 0.8013168 |

[Sequence list]

International application based on the International Patent Cooperation Treaty 19F01242W1JP20028406_2.app

**Claims**

1.  A bone regenerative agent comprising:

    gelatin-containing granules; and
    stem cells.

2.  The bone regenerative agent according to claim 1,

    wherein gelatin has repetitions of a sequence represented by Gly-X-Y, which is characteristic of collagen, where a plurality of Gly-X-Y's may be the same as or different from each other, and in the formula, X and Y each independently represent any of amino acid, and
    a molecular weight of the gelatin is 2 KDa or more and 100 KDa or less.

3.  The bone regenerative agent according to claim 1 or 2,
    wherein a size of one gelatin-containing granule is 100 $\mu$m or more and 2000 $\mu$m or less.

4.  The bone regenerative agent according to any one of claims 1 to 3,
    wherein the stem cells are mesenchymal stem cells, iPS cells, or ES cells.

**5.** The bone regenerative agent according to any one of claims 1 to 4,
wherein the stem cells are adipose-derived stem cells, bone marrow-derived stem cells, or dental pulp-derived stem cells.

**6.** The bone regenerative agent according to any one of claims 1 to 5,
wherein the number of stem cells per 1 mg of the gelatin-containing granules is $1.0 \times 10^3$ or more.

**7.** The bone regenerative agent according to any one of claims 1 to 6,
wherein 70% or more of the stem cells are attached to a surface or a pore of the surface of the gelatin-containing granules.

**8.** The bone regenerative agent according to any one of claims 1 to 7, which is used for a cleft jaw part.

FIG. 1

FIG. 2

$(mm^3)$

LARGE: $1 \times 10^5$, MEDIUM: $5 \times 10^4$, SMALL: $1 \times 10^4$

FIG. 3

# FIG. 4

VOLUME OF NEW BONE

mm³

1.5
1
0.5
0

GELATIN GRANULES
+ STEM CELLS
GELATIN GRANULES

0 WEEKS        4 WEEKS        8 WEEKS

FIG. 5

## INTERNATIONAL SEARCH REPORT

| International application No. |
| --- |
| PCT/JP2020/028406 |

**A. CLASSIFICATION OF SUBJECT MATTER**
A61K 45/00(2006.01)i; A61P 19/00(2006.01)i; C12N 5/0735(2010.01)i; C12N 5/0775(2010.01)i; C12N 5/10(2006.01)i; C12N 11/04(2006.01)i; A61K 35/28(2015.01)i; A61K 35/545(2015.01)i
FI: A61K35/28; A61K35/545; A61P19/00; A61K45/00; C12N5/10; C12N5/0735; C12N5/0775; C12N11/04 ZNA

According to International Patent Classification (IPC) or to both national classification and IPC

**B. FIELDS SEARCHED**

Minimum documentation searched (classification system followed by classification symbols)
A61K45/00; A61P19/00; C12N5/0735; C12N5/0775; C12N5/10; C12N11/04; A61K35/28; A61K35/545

Documentation searched other than minimum documentation to the extent that such documents are included in the fields searched
Published examined utility model applications of Japan          1922-1996
Published unexamined utility model applications of Japan          1971-2020
Registered utility model specifications of Japan          1996-2020
Published registered utility model applications of Japan          1994-2020

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2016/148245 A1 (FUJIFILM CORPORATION) 22.09.2016 (2016-09-22) claims, paragraphs [0027]-[0030], [0045], [0055] | 1-8 |
| X | JP 2003-260123 A (ED. GEISTLICH SOEHNE AG FUER CHEMISCHE INDUSTRIE) 16.09.2003 (2003-09-16) claims, paragraphs [0089], [0114], [0115] | 1-8 |
| X | JP 2000-508911 A (OSIRIS THERAPEUTICS, INC.) 18.07.2000 (2000-07-18) claims, pp. 19, 20 | 1-8 |
| X<br>A | LI, Xuewen, et al., "Synthesis and Evaluation of BMMSC-seeded BMP-6/nHAG/GMS Scaffolds for Bone Regeneration", Int. J. Med. Sci., 2016, vol. 16, no. 7, pp. 1007-1017, in particular, abstract, Materials and methods, fig. 2., 6. | 1, 2, 4, 5, 8<br>3, 6 |

☐ Further documents are listed in the continuation of Box C.          ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 16 September 2020 (16.09.2020) | 29 September 2020 (29.09.2020) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**
Information on patent family members

| International application no. |
|---|
| PCT/JP2020/028406 |

| Patent Documents referred in the Report | Publication Date | Patent Family | Publication Date |
|---|---|---|---|
| WO 2016/148245 A1 | 22 Sep. 2016 | US 2018/0064849 A1 claims, paragraphs [0061]-[0064], [0088], [0102], [0103] EP 3272370 A1 | |
| JP 2003-260123 A | 16 Sep. 2003 | US 2003/0180263 A1 claims, paragraphs [0047], [0102], [0146]-[0149] EP 1338291 A2 | |
| JP 2000-508911 A | 18 Jul. 2000 | WO 1997/040137 A1 claims, pp. 15, 16 US 2003/0031695 A1 EP 906415 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011027850 A
- WO 2016148245 A
- EP 1014176 B
- US 6992172 B
- WO 200485473 A
- WO 2008103041 A
- WO 2017170342 A
- EP 1014176 A2

**Non-patent literature cited in the description**

- Pathological Physiology. Nagai Publishing Co., Ltd, 1990, vol. 9, 527
- **MAXEY, C. R.** Phitogr. Gelatin. Academic, 1976, vol. 2
- *Pharmaceutical Bulletin,* 1954, vol. 2 (2), 163-173
- *Fields of Chemistry,* 1957, vol. 11 (10), 719-725
- *Fragrance Journal,* 1981, vol. 50, 79-82
- **YOSHIO KODA et al.** New Edition Organic Conceptual Diagram -Basics and Applications. Sankyo Publishing, 2008
- Protein Identification and Analysis Tools on the ExPASy Server. **GASTEIGER E. ; HOOGLAND C. ; GATTIKER A. ; DUVAUD S. ; WILKINS M. R. ; APPEL R. D. ; BAIROCH A.** The Proteomics Protocols Handbook. Humana Press, 2005, 571-607
- **GASTEIGER E. ; GATTIKER A. ; HOOGLAND C. ; IVANYI I. ; APPEL R.D. ; BAIROCH A.** ExPASy: the proteomics server for in-depth protein knowledge and analysis. *Nucleic Acids Res.,* 2003, vol. 31, 3784-3788

```
SEQUENCE LISTING
<110>  FUJIFILM Corporation
<120>  Biograft material
<130>\201@19F01242W1
<160>  10
<170>  PatentIn version 3.5
<210>  1
<211>  571
<212>  PRT
<213>  Recombinant
<400>  1
Gly Ala Pro Gly Ala Pro Gly Leu Gln Gly Ala Pro Gly Leu Gln Gly
1               5                   10                  15
Met Pro Gly Glu Arg Gly Ala Ala Gly Leu Pro Gly Pro Lys Gly Glu
                20                  25                  30
Arg Gly Asp Ala Gly Pro Lys Gly Ala Asp Gly Ala Pro Gly Ala Pro
                35                  40                  45
Gly Leu Gln Gly Met Pro Gly Glu Arg Gly Ala Ala Gly Leu Pro Gly
        50                  55                  60
Pro Lys Gly Glu Arg Gly Asp Ala Gly Pro Lys Gly Ala Asp Gly Ala
65                  70                  75                  80
Pro Gly Lys Asp Gly Val Arg Gly Leu Ala Gly Pro Ile Gly Pro Pro
                85                  90                  95
Gly Glu Arg Gly Ala Ala Gly Leu Pro Gly Pro Lys Gly Glu Arg Gly
                100                 105                 110
Asp Ala Gly Pro Lys Gly Ala Asp Gly Ala Pro Gly Lys Asp Gly Val
        115                 120                 125
Arg Gly Leu Ala Gly Pro Ile Gly Pro Pro Gly Pro Ala Gly Ala Pro
        130                 135                 140
Gly Ala Pro Gly Leu Gln Gly Met Pro Gly Glu Arg Gly Ala Ala Gly
145                 150                 155                 160
Leu Pro Gly Pro Lys Gly Glu Arg Gly Asp Ala Gly Pro Lys Gly Ala
                165                 170                 175
Asp Gly Ala Pro Gly Lys Asp Gly Val Arg Gly Leu Ala Gly Pro Pro
                180                 185                 190
Gly Ala Pro Gly Leu Gln Gly Ala Pro Gly Leu Gln Gly Met Pro Gly
                195                 200                 205
Glu Arg Gly Ala Ala Gly Leu Pro Gly Pro Lys Gly Glu Arg Gly Asp
        210                 215                 220
Ala Gly Pro Lys Gly Ala Asp Gly Ala Pro Gly Ala Pro Gly Leu Gln
225                 230                 235                 240
Gly Met Pro Gly Glu Arg Gly Ala Ala Gly Leu Pro Gly Pro Lys Gly
                245                 250                 255
Glu Arg Gly Asp Ala Gly Pro Lys Gly Ala Asp Gly Ala Pro Gly Lys
                260                 265                 270
Asp Gly Val Arg Gly Leu Ala Gly Pro Ile Gly Pro Pro Gly Glu Arg
        275                 280                 285
Gly Ala Ala Gly Leu Pro Gly Pro Lys Gly Glu Arg Gly Asp Ala Gly
        290                 295                 300
Pro Lys Gly Ala Asp Gly Ala Pro Gly Lys Asp Gly Val Arg Gly Leu
305                 310                 315                 320
Ala Gly Pro Ile Gly Pro Pro Gly Pro Ala Gly Ala Pro Gly Ala Pro
                325                 330                 335
Gly Leu Gln Gly Met Pro Gly Glu Arg Gly Ala Ala Gly Leu Pro Gly
        340                 345                 350
Pro Lys Gly Glu Arg Gly Asp Ala Gly Pro Lys Gly Ala Asp Gly Ala
        355                 360                 365
Pro Gly Lys Asp Gly Val Arg Gly Leu Ala Gly Pro Pro Gly Ala Pro
        370                 375                 380
Gly Leu Gln Gly Ala Pro Gly Leu Gln Gly Met Pro Gly Glu Arg Gly
385                 390                 395                 400
Ala Ala Gly Leu Pro Gly Pro Lys Gly Glu Arg Gly Asp Ala Gly Pro
                405                 410                 415
```

```
Lys Gly Ala Asp Gly Ala Pro Gly Ala Pro Gly Leu Gln Gly Met Pro
            420             425             430
Gly Glu Arg Gly Ala Ala Gly Leu Pro Gly Pro Lys Gly Glu Arg Gly
            435             440             445
Asp Ala Gly Pro Lys Gly Ala Asp Gly Ala Pro Gly Lys Asp Gly Val
    450             455             460
Arg Gly Leu Ala Gly Pro Ile Gly Pro Pro Gly Glu Arg Gly Ala Ala
465             470             475             480
Gly Leu Pro Gly Pro Lys Gly Glu Arg Gly Asp Ala Gly Pro Lys Gly
            485             490             495
Ala Asp Gly Ala Pro Gly Lys Asp Gly Val Arg Gly Leu Ala Gly Pro
            500             505             510
Ile Gly Pro Pro Gly Pro Ala Gly Ala Pro Gly Ala Pro Gly Leu Gln
            515             520             525
Gly Met Pro Gly Glu Arg Gly Ala Ala Gly Leu Pro Gly Pro Lys Gly
    530             535             540
Glu Arg Gly Asp Ala Gly Pro Lys Gly Ala Asp Gly Ala Pro Gly Lys
545             550             555             560
Asp Gly Val Arg Gly Leu Ala Gly Pro Pro Gly
                565             570
```

<210> 2
<211> 4
<212> PRT
<213> Artificial Sequence
<220>
<223> Description of Artificial Sequence: adhesive sequence
<400> 2

Arg Glu Asp Val
1

<210> 3
<211> 5
<212> PRT
<213> Artificial Sequence
<220>
<223> Description of Artificial Sequence: adhesive sequence
<400> 3

Tyr Ile Gly Ser Arg
1               5

<210> 4
<211> 5
<212> PRT
<213> Artificial Sequence
<220>
<223> Description of Artificial Sequence: adhesive sequence
<400> 4

Pro Asp Ser Gly Arg
1               5

<210> 5
<211> 7
<212> PRT
<213> Artificial Sequence
<220>
<223> Description of Artificial Sequence: adhesive sequence
<400> 5

Arg Tyr Val Val Leu Pro Arg
1               5

<210> 6
<211> 6
<212> PRT
<213> Artificial Sequence
<220>
<223> Description of Artificial Sequence: adhesive sequence
<400> 6

```
Leu Gly Thr Ile Pro Gly
1               5
<210>  7
<211>  10
<212>  PRT
<213>  Artificial Sequence
<220>
<223>  Description of Artificial Sequence: adhesive sequence
<400>  7
Arg Asn Ile Ala Glu Ile Ile Lys Asp Ile
1               5                   10
<210>  8
<211>  5
<212>  PRT
<213>  Artificial Sequence
<220>
<223>  Description of Artificial Sequence: adhesive sequence
<400>  8
Ile Lys Val Ala Val
1               5
<210>  9
<211>  4
<212>  PRT
<213>  Artificial Sequence
<220>
<223>  Description of Artificial Sequence: adhesive sequence
<400>  9
Asp Gly Glu Ala
1
<210>  10
<211>  4
<212>  PRT
<213>  Artificial Sequence
<220>
<223>  Description of Artificial Sequence: adhesive sequence
<400>  10
Glu Arg Gly Asp
1
```